# EUROPÄISCHE PATENTSCHRIFT

(11) **EP 0 453 951 B1**
(45) Veröffentlichungstag und Bekanntmachung des Hinweises auf die Patenterteilung: **21.09.1994**
(21) Anmeldenummer: 91106180.2
(22) Anmeldetag: 18.04.1991
(51) Int. Cl.: C07D 307/89, B01J 8/02, B01J 23/22

(54) **Verfahren zur Herstellung von Phthalsäureanhydrid aus o-Xylol**
Process for the preparation of phthalic anhydride from O-xylene
Procédé de préparation d'anhydride phthalique à partir d'O-xylène

(30) Priorität: 24.04.1990 DE 4013051
(43) Veröffentlichungstag der Anmeldung: 30.10.1991
(73) Patentinhaber: BASF Aktiengesellschaft, 67063 Ludwigshafen (DE)
(72) Erfinder: Aichinger, Heinrich, Dr., W-6800 Mannheim 1 (DE); Ruppel, Wilhelm, Dr., W-6830 Schwetzingen (DE); Seubert, Rolf, Dr., W-6710 Frankenthal (DE); Boehning, Karl-Heinz, Dr., W-6700 Ludwigshafen (DE); Scheidmeir, Walter, Dr., W-6703 Limburgerhof (DE); Schmidt, Johannes, Dr., W-6700 Ludwigshafen (DE); Schwarzmann, Matthias, Dr., W-6703 Limburgerhof (DE)

(56) Entgegenhaltungen:
- EP-A- 0 061 018
- EP-A- 0 264 747
- DE-A- 2 212 947
- DE-A- 2 830 765
- DE-A- 3 045 624
- DE-C- 2 201 528
- DE-C- 2 546 268
- DE-C- 2 547 624
- US-A- 4 835 126
- Chemical Abstracts, band 90, nr. 20, 14 Mai 1979,Columbus, Ohio, USA, CALDERBANK P. H. "The prediction of the performance of pacted-bed calalytic reactors in the air oxidation of o-xylene" Seite 340, Spalte 2, Zusammenfassung-Nr. 157 625p & Rev. Port. Quim. 1976, 18(1-4), 169-74.

## Beschreibung

Die Erfindung betrifft ein vorteilhaftes Verfahren zur Herstellung von Phthalsäureanhydrid durch katalytische Gasphasenoxidation von o-Xylol unter Verwendung von Vanadinpentoxid, Titandioxid, Antimon, Rubidium und/oder Cäsium und/oder Phosphor enthaltenden Trägerkatalysatoren mit 2 oder mehreren Reaktionszonen mit erhöhter Salzbadtemperatur in der ersten Reaktionszone gegenüber der in der zweiten oder folgenden Reaktionszonen.

Bekanntlich wird Phthalsäureanhydrid nach großtechnischen Verfahren durch katalytische Luftoxidation von o-Xylol in Festbettröhrenreaktoren hergestellt. Als Katalysatoren sind für diese Verfahren insbesondere Trägerkatalysatoren geeignet, die aus einem z.B. kugelförmigen inerten Träger und der darauf in dünner Schicht aufgebrachten katalytisch wirksamen Masse aus Vanadinpentoxid und Titandioxid bestehen. Solche Katalysatoren werden z.B. in der DE-A 14 42 590 beschrieben. Man hat auch schon Trägerkatalysatoren verwendet, deren katalytische Masse mit Phosphor (DE-A 17 69 998) bzw. mit Rubidium und/oder Cäsium (DE-A 24 36 009 und DE-A 25 47 624) dotiert ist.

Bei diesen bekannten Verfahren verfährt man im allgemeinen so, daß man ein Gemisch aus einem Sauerstoff enthaltenden Trägergas, wie Luft, und dem zu oxidierenden Kohlenwasserstoff durch eine Vielzahl der in dem Reaktor angeordneten Rohre leitet, in welchen sich der Katalysator befindet. Zur Temperaturregelung sind die Rohre mit einer Salzschmelze umgeben, in der man eine Temperatur von 350 bis 420°C einhält. Bei dieser Arbeitsweise werden störende Nebenprodukte gebildet, die sich von dem gewünschten Phthalsäureanhydrid nur schwer abtrennen lassen und die die Qualität des Phthalsäureanhydrids beeinträchtigen. Es ist dies bei der Phthalsäureanhydridherstellung aus o-Xylol vor allem Phthalid.

Die Bildung dieser Nebenprodukte fällt umso höher aus, je mehr die Beladung der Luft mit dem zu oxidierenden Kohlenwasserstoff gesteigert wird. Für eine wirtschaftliche Herstellung sind aber hohe Beladungen der Luft mit den zu oxidierenden Kohlenwasserstoffen erwünscht. Unter hohen Beladungen versteht man Beladungen, die über die untere Explosionsgrenze des Gemischs aus Luft und Kohlenwasserstoff hinausgehen, wie Beladungen von 44 bis 100 g o-Xylol je m³ Luft.

Die Entstehung der Nebenprodukte läßt sich z.B. dadurch zurückdrängen, daß man die Oxidation bei geringeren Gasdurchsätzen (längere Verweilzeit) oder niedrigeren Kohlenwasserstoffbeladungen der Luft durchführt. Dabei sinken jedoch die Ausbeuten an Phthalsäureanhydrid und die Reaktordurchsätze.

Eine Verbesserung des Verfahrens zur Herstellung von Phthalsäureanhydrid durch Luftoxidation von o-Xylol stellte daher der Einbau von mindestens 2 verschiedenen Katalysatoren pro Reaktionsrohr dar (DE-A 25 46 268). Bei diesem Verfahren wird bei Temperaturen von 350 bis 500°C o-Xylol mit Luft über ein Katalysatorbett geleitet, in dem der Katalysator der ersten 25 bis 50 Volumenprozent des Katalysatorvolumens in Strömungsrichtung des Gemisches aus o-Xylol und Luft in der aktiven Masse Rubidium oder Cäsium, aber keinen Phosphor und der restliche Katalysator in der aktiven Masse Phosphor, aber kein Rubidium oder Cäsium enthält. Nach diesem Verfahren wird die katalytische Oxidation der Kohlenwasserstoffe z.B. auf an sich bekannte Weise in einem Röhrenreaktor mit Salzbadkühlung bei Temperaturen von 350 bis 500°C, vorzugsweise 350 bis 400°C des Salzbades, durchgeführt. Die von dem Salzbad umgebenen Rohre des Reaktors, die einen Durchmesser von 18 bis 40 mm und eine Länge von 2 bis 3,5 m aufweisen, sind mit dem Katalysator gefüllt. Der Katalysator ist ein Trägerkatalysator, der aus einem katalytisch inerten Träger mit einem Durchmesser von 3 bis 13 mm und der darauf in dünner Schicht aufgebrachten katalytischen Masse besteht. Der Träger hat z.B. die Gestalt einer Kugel oder vorteilhaft Ringform. Er besteht aus gesinterten oder geschmolzenen Silikaten, Porzellan, Tonerde, Siliziumcarbid oder Quarz. Die katalytisch aktive Masse, die den Träger mit einer Schichtdicke von 0,05 bis 1 mm überzieht, enthält z.B. 1 bis 30 Gew.-% Vanadinpentoxid und 70 bis 99 Gew.-% Titandioxid. Sie kann gegebenenfalls noch geringe Mengen, z.B. bis zu 5 Gew.-%, bezogen auf die katalytische Masse, an Antimon, Zirkon oder Zinn, Phosphor, Rubidium oder Cäsium, z.B. in Form ihrer Oxide enthalten. Die aktive Masse macht etwa 3 bis 50 Gew.-% des fertigen Trägerkatalysators aus.

Bei diesem Verfahren befinden sich 2 oder mehrere verschiedene Katalysatoren in einem bezüglich der Salzkreisläufe und damit hinsichtlich der Temperaturführung ungeteilten Rohrbündelreaktor, wobei die Rohre mit einer Salzschmelze umgeben sind, in der man eine bestimmte einheitliche Temperatur im Bereich von 350 bis 420°C einhält.

Seit Reaktoren mit zwei- oder mehrstufigem Salzbad und individueller Rohrdimensionierung als kompakte Einheit großtechnisch realisierbar (z.B. DE-A 22 01 528) geworden sind, kann ein Reaktionsrohr in zwei oder mehrere Abschnitte eingeteilt werden mit jeweils eigener Salzbadtemperatur, eigenem Rohrdurchmesser und eigenem Katalysator.

Dementsprechend wurden bereits Reaktionsführungen mit 2 Salzkreisläufen für die Oxidation von n-Butan zu Maleinsäureanhydrid (Wellauer et al., Chem. Eng. Sci. 41, 765 (1986) und die Oxidation von o-Xylol zu Phthalsäureanhydrid (DE-A 28 30 765) vorgeschlagen. In beiden Fällen soll jedoch zwingend die Temperatur am Reaktorausgang gegenüber der Temperatur am Reaktoreingang erhöht werden, d.h. die Salzbadtemperatur der ersten Reaktionszone ist niedriger als die der zweiten Zone. Die Nacharbeitung des Verfahrens dieser Patentschrift unter Verwendung handelsüblicher Katalysatoren ergab jedoch keine Verbesserung gegenüber einer einstufigen Fahrweise.

Überraschenderweise wurde nun gefunden, daß man verbesserte Ausbeuten mit einem Verfahren zur Herstellung von Phthalsäureanhydrid durch Oxidation von o-Xylol im Rohrbündelreaktor in mindestens zwei in Strömungsrichtung aufeinanderfolgenden Reaktionszonen mit getrennter Salzbadkühlung an mit katalytisch aktiven, Vanadinpentoxid, Titandioxid, Antimon, Rubidium und/oder Cäsium und/oder Phosphor enthaltenden Massen beschichteten Trägerkatalysatoren, bei dem man das o-Xylol mit Luft über den Katalysator bei Temperaturen von 320 bis 380°C leitet, erzielt, wenn die Salzbadtemperatur der ersten Reaktionszone in Strömungsrichtung des Reaktionsgemisches mit 30 bis 75 Volumenprozent des gesamten Katalysatorvolumens um 2 bis 20°C höher gehalten wird als die Salzbadtemperatur der verbleibenden Reaktionszone oder Reaktionszonen mit 25 bis 70 Volumenprozent des gesamten Katalysatorsystems, wobei die Salzbadtemperatur für die erste Reaktionszone innerhalb des genannten Bereiches von 320 bis 380°C so eingestellt wird, daß nahezu vollständiger Umsatz, z.B. mehr als 99,5 %iger Umsatz, des o-Xylols bei maximaler Ausbeute erfolgt.

Gemäß einer bevorzugten Ausführungsform der Erfindung verwendet man 2 durch getrennte Salzbäder gekühlte Reaktionszonen, wobei die Salzbadtemperatur des ersten Salzbades bevorzugt 2 bis 15°C und insbesondere 5 bis 15°C über der des zweiten Salzbades liegt.

Die für die Reaktionszonen verwendeten Katalysatoren sind z.B. die in den eingangs genannten Patentschriften (auf die hiermit Bezug genommen wird) beschriebenen, an sich bekannten Vanadiumoxid auf einem unporösen inerten Träger enthaltenden Katalysatoren. Werden nach der weiter bevorzugten Arbeitsweise 2 verschiedene Katalysatoren in den aufeinanderfolgenden Reaktionszonen verwendet, so enthält der Katalysator in der ersten Reaktionszone zweckmäßig zusätzlich Rubidium und/oder Cäsium wie in der DE-A 24 36 009 beschrieben, der Katalysator der zweiten Zone vorteilhaft Phosphor gemäß DE-A 17 69 998. Der Einfachheit halber wird im Hinblick auf weitere Einzelheiten auf die beiden genannten Patentschriften Bezug genommen.

Wie bereits erwähnt, ist das erfindungsgemäße Verfahren besonders vorteilhaft, wenn die Beladung der Luft mit o-Xylol über die untere Explosionsgrenze, d.i. über 44 g/m³ Luft hinausgeht und besonders, wenn sie 60 g/m³ und mehr beträgt.

### Beispiele

### Herstellung des Katalysators

Der Katalysator I besteht aus Steatitkugeln des Durchmessers 4,3 mm und einer aktiven Masse mit der Schichtdicke 0,1 mm. Die nach den Angaben der DE-A 25 47 624 Beispiele aufgebrachte aktive Masse besteht aus 7,00 Gew.-% Vanadinpentoxid, 2,5 Gew.-% Antimonoxid und 0,16 Gew.-% Rubidium. Die restliche aktive Masse besteht aus Titandioxid (Anatas).

### Herstellung des Katalysators II

Der Katalysator II besteht aus Steatitkugeln des Durchmessers 4,3 mm und einer aktiven Masse mit der Schichtdicke 0,1 mm. Die nach DE-PS 25 47 624 aufgebrachte aktive Masse besteht aus 7,0 Gew.-% Vanadinpentoxid, 2,5 Gew.-% Antimonoxid und 0,5 Gew.-% Phosphor. Die restliche aktive Masse besteht aus Titandioxid (Anatas).

### Oxidation

0,75 m des Katalysators II und darüber, zum Rohreingang hin, 0,90 m des Katalysators I werden in einen 1,95 m langen, abschnittsweise mit getrenntem Salzbad ausgestatteten Rohrreaktor mit einer lichten Weite der Rohre von 15 mm eingefüllt. Durch das Rohr werden bei Katalysatorbelastungen (GHSV) von 2850⁻¹ Reaktionsmischungen mit Beladungen von etwa 60 g o-Xylol/Nm³ Luft über Katalysator I und II (in dieser Reihenfolge) geleitet.

### Vergleichsbeispiel 1

In dem oben beschriebenen Reaktor wurden die Katalysatoren I und II bei nur einer Reaktortemperatur betrieben. Dabei wurden die in folgender Tabelle zusammengefaßten Ergebnisse erhalten.

| Reaktortemperatur [°C] | Beladung [g] o-Xylol/Nm³ Luft | Ausbeute Phthalsäureanhydrid (PSA) [mol-%] (nach Destillation) |
|---|---|---|
| 358 | 60 | 76,0 |
| 357 | 60 | 76,5 |
| 356 | 60 | 76,4 |

### Vergleichsbeispiel 2

In dem oben beschriebenen Reaktor wurden die Katalysatoren I und II bei jeweils unterschiedlicher Salzbadtemperatur betrieben, wobei die Salzbadtemperatur, mit der der Katalysator I gekühlt wird, niedriger gehalten wurde als die Salzbadtemperatur für Katalysator II. Dabei wurden die in folgender Tabelle zusammengefaßten Ergebnisse erhalten.

| Reaktortemperatur [°C] | | Beladung [g] o-Xylol/Nm³ Luft | Ausbeute PSA [mol-%] (nach Destillation) |
|---|---|---|---|
| Katalys. I | Katalys. II | | |
| 354 | 356 | 60 | 72,2 |
| 354 | 355 | 60 | 72,6 |
| 353 | 355 | 60 | 71,3 |
| 350 | 356 | 60 | kein vollständiger Xylolumsatz |

### Ausführungsbeispiel

In dem oben beschriebenen Reaktor wurden die Katalysatoren I und II bei jeweils unterschiedlicher Salzbadtemperatur betrieben, wobei die Salzbadtemperatur, mit der der Katalysator I gekühlt wird, höher gehalten wurde als die Salzbadtemperatur für Katalysator II. Dabei wurden die in folgender Tabelle zusammengefaßten Ergebnisse erhalten. Die Ausbeuten an Phthalsäureanhydrid waren höher als die im Vergleichsbeispiel erhaltenen Ausbeuten beim Betrieb beider Katalysatoren bei einer einheitlichen Salzbadtemperatur.

| Reaktortemperatur [°C] | | Beladung [g] o-Xylol/Nm³ Luft | Ausbeute PSA [mol-%] (nach Destillation) |
|---|---|---|---|
| Katalys. I | Katalys. II | | |
| 357 | 350 | 60 | 78,2 |
| 358 | 345 | 60 | 78,2 |
| 359 | 345 | 60 | 77,5 |
| 359 | 344 | 60 | 78,3 |
| 359 | 343 | 60 | 78,6 |
| 359 | 342 | 60 | 78,7 |

## Patentansprüche

1. Verfahren zur Herstellung von Phthalsäureanhydrid durch Oxidation von o-Xylol im Rohrbündelreaktor in mindestens zwei in Strömungsrichtung aufeinanderfolgenden Reaktionszonen mit getrennter Salzbadkühlung an mit katalytisch aktiven, Vanadinpentoxid, Titandioxid, Antimon, Rubidium und/oder Cäsium und/oder Phosphor enthaltenden Massen beschichteten Trägerkatalysatoren, wobei man das o-Xylol mit Luft mit einer Beladung von mindestens 60 g o-Xylol pro m³ Luft über den Katalysator bei Temperaturen von 320 bis 380°C leitet, dadurch gekennzeichnet, daß die Salzbadtemperatur der ersten Reaktionszone in Strömungsrichtung des Reaktionsgemisches mit 30 bis 75 Volumenprozent des gesamten Katalysatorvolumens um 2 bis 20°C höher gehalten wird als die Salzbadtemperatur der verbleibenden Reaktionszone oder Reaktionszonen mit 25 bis 70 Volumenprozent des gesamten Katalysatorvolumens, wobei die Salzbadtemperatur für die erste Reaktionszone innerhalb des genannten Bereiches von 320 bis 380°C so eingestellt wird, daß nahezu vollständiger Umsatz des o-Xylols bei maximaler Ausbeute erfolgt.

2. Verfahren nach Anspruch 1, dadurch gekennzeichnet, daß man einen Reaktor mit 2 Reaktionszonen, die durch getrennte Salzbäder gekühlt werden, verwendet.

3. Verfahren nach Anspruch 1, dadurch gekennzeichnet, daß man in der ersten und der zweiten oder folgenden Reaktionszone den gleichen Katalysator verwendet.

4. Verfahren nach Anspruch 1, dadurch gekennzeichnet, daß man in der ersten und zweiten oder den folgenden Reaktionszonen verschiedene Katalysatoren verwendet.

5. Verfahren nach Anspruch 4, dadurch gekennzeichnet, daß der Katalysator der zweiten Reaktionszone zusätzlich Phosphor enthält.

6. Verfahren nach Anspruch 4, dadurch gekennzeichnet, daß der Katalysator der ersten Reaktionszone zusätzlich Rubidium und/oder Cäsium enthält.

7. Verfahren nach Anspruch 1, dadurch gekennzeichnet, daß man das Verfahren mit einer Beladung des Reaktionsgemisches von mindestens 60 g o-Xylol pro m³ Luft durchführt.

## Claims

1. A process for the preparation of phthalic anhydride by oxidation of o-xylene in a tube-bundle reactor in at least two reaction zones in succession in the direction of flow and having separate salt bath cooling, over supported catalysts coated with catalytically active materials containing vanadium pentoxide, titanium dioxide, antimony, rubidium and/or caesium and/or phosphorus, the o-xylene being passed with air with a loading of at least 60 g of o-xylene per m³ of air over the catalyst at from 320 to 380°C, wherein the salt bath temperature of the first reaction zone in the direction of flow of the reaction mixture, accounting for from 30 to 75% by volume of the total catalyst volume, is kept from 2 to 20°C higher than the salt bath temperature of the remaining reaction zone or reaction zones, accounting for from 25 to 70% by volume of the total catalyst volume, the salt bath temperature for the first reaction zone being brought within the stated range of from 320 to 380°C, resulting in virtually complete conversion of the o-xylene with maximum yield.

2. A process as claimed in claim 1, wherein a reactor having 2 reaction zones which are cooled by separate salt baths is used.

3. A process as claimed in claim 1, wherein the same catalyst is used in the first and the second or subsequent reaction zone.

4. A process as claimed in claim 1, wherein different catalysts are used in the first and second or the subsequent reaction zones.

5. A process as claimed in claim 4, wherein the catalyst of the second reaction zone additionally contains phosphorus.

6. A process as claimed in claim 4, wherein the catalyst of the first reaction zone additionally contains rubidium and/or caesium.

7. A process as claimed in claim 1, wherein the process is carried out with a loading of the reaction mixture of at least 60 g of o-xylene per m³ of air.

## Revendications

1. Procédé de préparation de l'anhydride phtalique par l'oxydation du o-xylène dans un réacteur à faisceau de tubes dans au moins deux zones réactionnelles qui se font suite en direction du courant, avec refroidissement par bain de sel séparé, sur des catalyseurs à support, revêtus de masses catalytiquement actives, contenant du pentoxyde de vanadium, du dioxyde de titane, de l'antimoine, du rubidium et/ou du césium et/ou du phosphore, conformément auquel on fait passer le o-xylène avec de l'air et sous une charge d'au moins 60 g de o-xylène par m³ d'air sur le catalyseur, à des températures de 320 à 380°C, caractérisé en ce que la température du bain de sel de la première zone de réaction en direction du courant du mélange de réaction avec 30 à 75% volumiques du volume total du catalyseur, a une valeur de 2 à 20°C supérieure à la température du bain de sel de la ou des zones réactionnelles subsistantes avec 25 à 70% volumiques du volume total du catalyseur, où la température du bain de sel pour la première zone réactionnelle est réglée à l'intérieur de la plage susmentionnée de 320 à 380°C en une manière telle qu'une conversion pratiquement complète du o-xylène se déroule avec un rendement maximal.

2. Procédé suivant la revendication 1, caractérisé en ce que l'on utilise un réacteur à deux zones réactionnelles, qui sont refroidies par des bains de sel séparés.

3. Procédé suivant la revendication 1, caractérisé en ce que l'on utilise le même catalyseur dans la première et la seconde ou les zones réactionnelles suivantes.

4. Procédé suivant la revendication 1, caractérisé en ce que l'on utilise des catalyseurs différents dans la première et la seconde ou les zones réactionnelles suivantes.

5. Procédé suivant la revendication 4, caractérisé en ce que le catalyseur de la seconde zone réactionnelle contient complémentairement du phosphore.

6. Procédé suivant la revendication 4, caractérisé en ce que le catalyseur de la première zone réactionnelle contient complémentairement du rubidium et/ou du césium.

7. Procédé suivant la revendication 1, caractérisé en ce que l'on entreprend le procédé avec une charge du mélange réactionnel d'au moins 60 g de o-xylène par m³ d'air.
